# EUROPEAN PATENT APPLICATION

(11) **EP 3 872 818 A2**
(43) Date of publication of application: **01.09.2021**
(21) Application number: 19890929.3
(22) Date of filing: 27.11.2019
(51) Int. Cl.: G16H 50/20, G16H 50/30, G16H 50/70, G16H 40/60

(54) **METHOD FOR PROVIDING DIAGNOSTIC SYSTEM USING SEMI-SUPERVISED LEARNING, AND DIAGNOSTIC SYSTEM USING SAME**

(30) Priority: 30.11.2018 KR 20180153152
(71) Applicant: Deep Bio Inc., Seoul 08394 (KR)
(72) Inventor: KIM, Sun Woo, Seongnam-si Gyeonggi-do 13599 (KR)
(74) Representative: Flügel Preissner Schober Seidel
(86) International application number: PCT/KR2019/016425
(87) International publication number: WO 2020/111754

(57) **Abstract**

A method for providing a diagnostic system using semi-supervised learning, and a system therefor are disclosed. The method for providing a diagnostic system using semi-supervised learning comprises: a step in which a diagnostic system learned through a neural network on the basis of supervised learning receives predetermined input data and outputs a diagnostic result for the input data; a step in which the diagnostic system generates automatic annotation training data including the input data annotated as the diagnostic result; and a step in which the diagnostic system performs a retraining process by using the generated automatic annotation training data.

## Description

### [Technical Field]

The present disclosure relates to a learning method using a neural network and a diagnosis system using the same, and more particularly, to a method and system capable of increasing performance by generating training data by automatically annotating data using a system (e.g., a system for diagnosing a disease) trained based on supervised learning and performing retraining on a system using training data automatically generated as described above, that is, automatically annotated.

### [Background Art]

A deep learning (e.g., a deep learning method using a convolution neural network (CNN)) technology using a neural network is widely used.

Attempts to perform a diagnosis of a disease by using such deep learning are gradually increased.

For example, one of major tasks performed in pathology or a pathology department is a task for performing a diagnosis for determining a state or symptom of a specific disease by reading a bio image of a patient. Such a diagnosis is a method dependent on experiences and knowledge of a healthcare worker experienced for a long period.

However, even in this diagnosis, with the development of machine learning, attempts to automate a task, such as recognizing or classifying images, using a computer system are actively made. In particular, attempts to automate a diagnosis performed by an experienced healthcare worker by using a neural network, that is, a kind of machine learning, is carried out.

In particular, a diagnosis through deep learning using a neural network (e.g., CNN) also includes a case where a characteristic of a disease factor not found by an experienced healthcare worker is found out in an image in that a desired solution is derived by autonomously finding out characteristic features through learning without simply automating experiences and knowledge of an experienced healthcare worker as in a conventional technology.

In general, a diagnosis of a disease through a neural network using bio data (e.g., bio image) includes that an experienced healthcare worker annotates the bio data with a state of a specific disease (e.g., whether cancer has developed). The neural network is trained using such multiple annotated data as training data. That is, training data is annotated for training, and learning through the annotated training data is chiefly used. Such a learning method is called supervised learning.

However, such supervised learning requires multiple annotated training data for supervised learning. Furthermore, generating such multiple annotated training data is a process that requires high expenses and time because an experienced expert must perform the generation of the multiple annotated training data for a great deal of time.

Accordingly, there is a need for a learning method capable of slightly reducing time and costs consumed for a process of preparing training data through such annotation and also implementing a trained system having excellent performance (e.g., a system for diagnosing a disease).

### *Prior Art Document

### -Patent Document

Korean Patent No. 10-1818074 "ARTIFICIAL INTELLIGENCE BASED MEDICAL AUTO DIAGNOSIS AUXILIARY METHOD AND SYSTEM THEREFOR"

### [Disclosure]

### [Technical Problem]

An object of the present disclosure is to provide a method and system capable of increasing diagnosis performance of a diagnosis system by using, in retraining, results diagnosed by a system trained based on supervised learning in order to diagnose a disease.

Furthermore, an object of the present disclosure is to provide a method and system capable of effectively determining a reference threshold value of a numerical value, that is, a base when a diagnosis system determines a diagnosis result.

Furthermore, an object of the present disclosure is to provide a method and system capable of further improving performance by resetting such a reference threshold value of a numerical value at given timing after retraining.

### [Technical Solution]

A method of providing a diagnosis system using semi-supervised learning for achieving the technical object includes the steps of receiving, by a diagnosis system trained through a supervised learning-based neural network, given input data and outputting a diagnosis result of the input data, generating, by the diagnosis system, automated annotation training data including the input data annotated as the diagnosis result, and performing, by the diagnosis system, a retraining process by using the generated automated annotation training data.

The step of generating, by the diagnosis system, the automated annotation training data including the input data annotated as the diagnosis result may include including the input data in the automated annotation training data when a numerical value which is based on the diagnosis result and indicates a probability of the diagnosis result is a given threshold value or more.

The method of providing a diagnosis system using semi-supervised learning may further include the steps of testing performance of the diagnosis system after performing the retraining process while changing the threshold value and determining a reference threshold value based on the results of the test.

The method of providing a diagnosis system using semi-supervised learning may further include the steps of generating, by the diagnosis system, the automated annotation training data by using a reference threshold value, performing the retraining process by using the generated automated annotation training data, and changing the reference threshold value after performing the retraining process.

The diagnosis system may output, as the diagnosis result, any one of a plurality of diagnoses including a first determination and a second determination with respect to the input data. The automated annotation training data may include a predetermined number or more of input data annotated as a first diagnosis and a predetermined number or more input data annotated as a second diagnosis.

The input data may be bio data, and the diagnosis result may be at least any one of classes according to whether a disease has developed or a progress state of the disease.

The method may be implemented a computer program installed in a data processing apparatus.

A diagnosis system for achieving the technical object includes a processor and a storage device in which a program executed by the processor is stored. The program is stored in the storage device, enables a neural network trained based on supervised learning to receive given input data and output a diagnosis result of the input data, generates automated annotation training data including the input data annotated as the output diagnosis result, and performs retraining process of the neural network by using the generated automated annotation training data.

The program may include the input data in the automated annotation training data when a numerical value which is based on the diagnosis result and indicates a probability of the diagnosis result is a given threshold value or more, may test performance of the diagnosis system after performing the retraining process while changing the threshold value, and may determine a reference threshold value based on the results of the test.

The program may generate the automated annotation training data by using a reference threshold value, may perform the retraining process by using the generated automated annotation training data, and may change the reference threshold value after performing the retraining process.

The program may output, as the diagnosis result, any one of a plurality of diagnoses including a first determination and a second determination with respect to the input data. The automated annotation training data may include a predetermined number or more of input data annotated as a first diagnosis, and a predetermined number or more input data annotated as a second diagnosis.

### [Advantageous Effects]

According to the technical spirit of the present disclosure, there is an effect in that an annotation process requiring lots of costs and time for, in particular, annotation can be effectively performed using, in retraining, results diagnosed by a system trained based on supervised learning in order to diagnose a disease. Furthermore, there is an effect in that diagnosis performance of a diagnosis system can be improved through retraining.

Furthermore, there is an effect in that performance of a diagnosis system after retraining can be improved because a reference threshold value of a numerical value, that is, a base when the diagnosis system determines a diagnosis result, is effectively determined based on improved performance of retraining.

Furthermore, there is an effect in that performance can be further improved by resetting such a reference threshold value of a numerical value at given timing after retraining.

### [Description of Drawings]

A brief description of the drawings is provided so that the drawings cited in the detailed description of the present disclosure are sufficiently understood.
FIG. 1 is a diagram illustrating a schematic system configuration for implementing a method of providing a diagnosis system using semi-supervised learning according to the technical spirit of the present disclosure.
FIG. 2 is a diagram for describing a schematic configuration of a diagnosis system according to an embodiment of the present disclosure.
FIG. 3 is a diagram for describing a schematic diagnosis method of a neural network according to an embodiment of the present disclosure.
FIG. 4 is a diagram for describing a criterion for setting a reference threshold value according to an embodiment of the present disclosure.
FIG. 5 is a diagram for describing a method of resetting a reference threshold value according to an embodiment of the present disclosure.

### [Best Mode for Invention]

The present disclosure may be variously changed and may have various embodiments. Specific embodiments will be illustrated in the drawings and will be described in detail in the detailed description. It is however to be understood that the present disclosure is not intended to be limited to the specific embodiments and includes all changes, equivalents and substitutions included in the spirit and technical range of the present disclosure. In describing the present disclosure, a detailed description of a related known technology will be omitted if it is deemed to make the gist of the present disclosure unnecessarily vague.

Terms, such as "first" and "second", may be used to describe various elements, but the elements should not be restricted by the terms. The terms are used to only distinguish one element from the other element.

The terms used in this application are used to merely describe specific embodiments and are not intended to restrict the present disclosure. An expression of the singular number includes an expression of the plural number unless clearly defined otherwise in the context.

In this specification, it is to be understood that a term, such as "include" or "have", is intended to designate that a characteristic, a number, a step, an operation, an element, a part or a combination of them described in the specification is present, and does not exclude the presence or addition possibility of one or more other characteristics, numbers, steps, operations, elements, parts, or combinations of them in advance.

Furthermore, in this specification, if any one element "transmits" data to another element, this means that the one element may directly transmit the data to the another element or may transmit the data to the another element through at least another element. In contrast, if any one element "directly transmits" data to another element, this means that the data is transmitted the another element without the intervention of another element.

Hereinafter, the present disclosure is described in detail based on embodiments of the present disclosure with reference to the accompanying drawings. The same reference numerals described in drawings refer to the same elements.

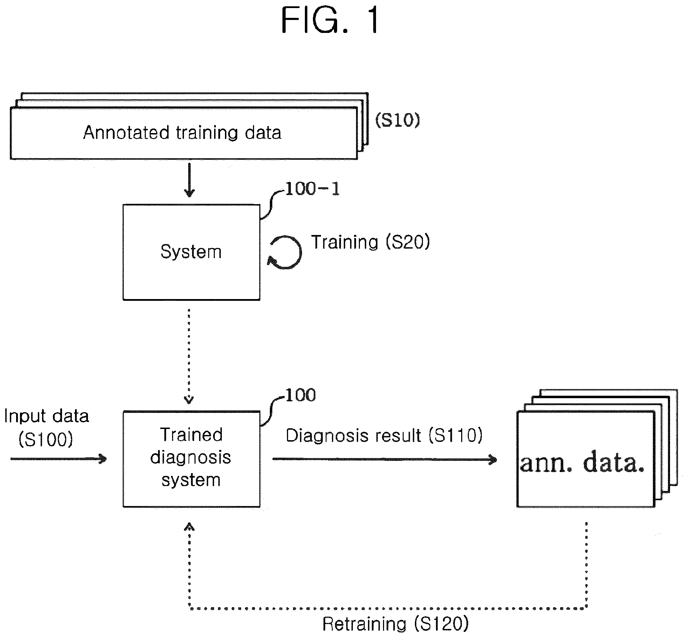

FIG. 1 is a diagram illustrating a schematic system configuration for implementing a method of providing a diagnosis system using semi-supervised learning according to the technical spirit of the present disclosure.

Referring to FIG. 1, in order to implement a method of providing a diagnosis system using semi-supervised learning according to the technical spirit of the present disclosure, a trained diagnosis system 100 may be implemented based on supervised learning.

According to the technical spirit of the present disclosure, the diagnosis system 100 may be a system trained based on supervised learning in a way to output a diagnosis result when receiving given input data.

For example, the diagnosis system 100 may be a system, which is trained based on supervised learning, classifies given bio data (e.g., bio image) into predetermined classes (e.g., classes according to whether a disease has developed or a degree of the progress of the disease) through a trained neural network when receiving the bio data, and outputs a corresponding result as a diagnosis result.

The diagnosis system 100 may be implemented using various data processing systems (e.g., a computer, a server, a smartphone or a dedicated device) if the diagnosis system can have only to perform a function defined in this specification.

The diagnosis system 100 may be trained using multiple training data. If a system 100-1 equipped with a given neural network receives the multiple training data (S10) and performs training (S20), the diagnosis system 100 may be implemented.

The training data may be data previously annotated so that a desired diagnosis system 100 outputs a diagnosis result. The annotation may be information corresponding to the diagnosis result output by the diagnosis system 100.

If the diagnosis system 100 is a system that simply receive input data (e.g., bio image) and outputs, as a diagnosis result, whether a disease has developed in the corresponding input data, the annotation may also mean labeling information on whether a disease has developed in each of multiple data (e.g., bio image).

Alternatively, if the diagnosis system 100 is a system that outputs a degree of progress state of a disease (e.g., a Gleason score in the case of prostate cancer) as a diagnosis result, the annotation may also mean labeling a degree of a progress state of a disease on each of multiple data (e.g., bio image).

In either case, a diagnosis result output by the diagnosis system 100 may be dependently defined based on annotated information.

As described above, performance of the diagnosis system 100 trained using annotated training data as described above may be dependent on the amount and quality of training data.

However, in particular, in order for the trained diagnosis system 100 to be used in the diagnosis of a disease, such annotation needs to be performed by an experienced healthcare worker who can diagnose a diagnosis result based on input data, and such annotation needs to be performed on each of a sufficiently large amount of training data.

Accordingly, in order to reduce such a problem, in the method of providing a diagnosis system using semi-supervised learning according to the technical spirit of the present disclosure, the diagnosis system 100 trained based on supervised learning to some extent may be implemented. Thereafter, a diagnosis result of the diagnosis system 100 may be set as an annotation result of input data corresponding to the diagnosis result.

For example, when the diagnosis system 100 already trained based on supervised learning receives specific input data (S100), the diagnosis system 100 may output a diagnosis result (S110).

Accordingly, the specific input data may be set as data annotated as the diagnosis result.

As described above, a diagnosis result is set as an annotation result of input data by the diagnosis system 100 trained based on supervised learning. Data annotated as the diagnosis result of the diagnosis system 100 is defined as automated annotation training data.

Such automated annotation training data may be used as training data for the retraining of the diagnosis system 100 (S120). That is, the diagnosis system 100 may perform retraining by using the automated annotation training data.

Meanwhile, all input data output as diagnosis results by the diagnosis system 100 may not be used as automated annotation training data. That is, the automated annotation training data, as will be described later, is data used for the retraining of the diagnosis system 100, and thus an annotation result of the automated annotation training data needs to be reliable.

Accordingly, when the diagnosis system 100 outputs a given diagnosis result for input data, the automated annotation training data may be limited to input data when diagnosis results are output at a given probability or more.

A neural network may be designed so that a numerical value corresponding to such a probability is output by a layer prior to the final layer of the neural network included in the diagnosis system 100. In an embodiment, the final layer of the neural network may be implemented to output the numerical value. In either case, at least one layer of the neural network included in the diagnosis system 100 may be designed and trained to output a numerical value, that is, a base that determines a diagnosis result before the final diagnosis result is output.

Accordingly, the diagnosis system 100 may output a diagnosis result (classify input data) as a first diagnosis result (e.g., a first class, such as whether a disease has developed or a degree of the progress state of the disease) when the numerical value is a predetermined threshold value or more, and may output the diagnosis result as a second diagnosis result (a second class of a plurality of classes capable of being classified as the diagnosis result) when the numerical value is less than the threshold value.

According to another embodiment, the diagnosis system 100 may separately define a threshold value, which is a criterion to be used as automated annotation training data, separately from a criterion for a numerical value, that is, a criterion for classifying a diagnosis result.

For example, when a numerical value is a given value (e.g., 0.8) or more, the first diagnosis result is output, but a threshold value which is a criterion to be used as automated annotation training data may be 0.85. That is, only input data having a threshold value of 0.85 or more, among input data classified as the first diagnosis result, may be used as the automated annotation training data.

In either case, the threshold value may become a criterion for determining whether to use input data as the automated annotation training data if the input data received by the diagnosis system 100 is classified as a given diagnosis result by the diagnosis system 100.

Furthermore, as described above, a threshold value which is a criterion to be used as automated annotation training data may be defined as a reference threshold value. Data to be included in the automated annotation training data may be determined based on the reference threshold value, and the determined automated annotation training data may be used for the retraining of the diagnosis system 100. Accordingly, the reference threshold value may have a great influence on performance of the diagnosis system 100 after the retraining.

In an embodiment, a numerical value, that is, a base for a diagnosis result, may correspond to a probability indicative of the precision of a first diagnosis result (e.g., a first class of a plurality of classes) among diagnosis results (e.g., the plurality of classes) which may be output by the diagnosis system 100. However, in an embodiment, the automated annotation training data may also need to include input data classified as another diagnosis result (e.g., another class not a first class of a plurality of classes) in addition to input data classified as a first diagnosis result. Accordingly, as a reference threshold value becomes higher, performance of the diagnosis system 100 after retraining may not be improved.

Accordingly, according to the technical spirit of the present disclosure, the reference threshold value may also be determined by the diagnosis system 100.

The diagnosis system 100 may set a plurality of threshold values, and may specify a plurality of automated annotation training data sets to be used for retraining based on the plurality of set threshold values, respectively. Each of the automated annotation training data sets may be specified to include a predetermined number of data or more annotated as a plurality of diagnosis results. In an embodiment, only data annotated as any one diagnosis result may be specified as the automated annotation training data. However, in general, if training data is prepared to have a similar percentage for each diagnosis result, a better advantageous effect can be obtained for learning performance. Accordingly, the automated annotation training data set may be defined so that data annotated each of a plurality of diagnosis results (i.e., classified classes) is included in a predetermined number of automated annotation training data or more.

Furthermore, the diagnosis system 100 may be retrained using each of the plurality of different training data sets. To this end, a plurality of neural networks identical with the diagnosis systems 100 may be prepared.

Furthermore, performance evaluation may be performed using a test data set previously defined for each of a plurality of retrained diagnosis systems 100. Furthermore, a threshold value having the best performance after retraining may be determined as a reference threshold value based on a result of the performance evaluation.

When such a reference threshold value is determined, the diagnosis system 100 may generate multiple automated annotation training data based on the reference threshold value. Furthermore, if a given number of automated annotation training data or more is generated (in this case, the given number may be greater than the number of automated annotation training data used to determine the reference threshold value), the diagnosis system 100 may perform retraining by using the automated annotation training data.

The diagnosis system 100 on which retraining has been performed may have better performance than the diagnosis system 100 before the retraining.

Furthermore, the diagnosis system 100 having the improved performance may be a system in which multiple parameters constituting a neural network have been changed compared to the diagnosis system 100 before the retraining. Accordingly, it may not be preferred to generate automated annotation training data for performing retraining by continuously using the existing reference threshold value. Accordingly, it may be preferred to change a criterion for classifying automated annotation training data to be used when retraining is performed again by performing a process of determining a reference threshold value on the diagnosis system 100 after the retraining as described above.

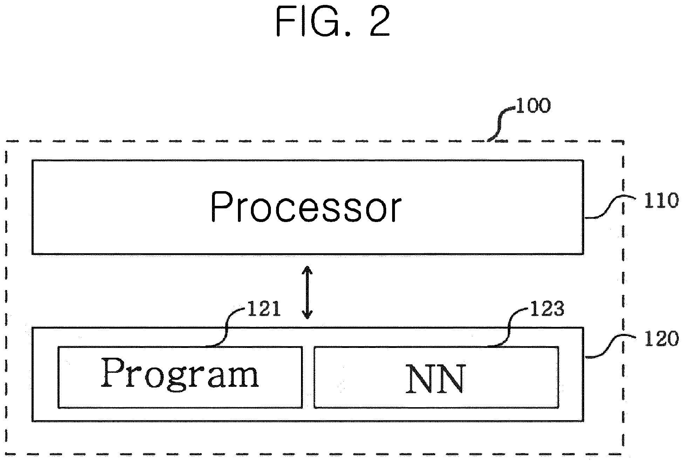

A schematic configuration of the diagnosis system 100 for implementing such a technical spirit may be the same as that illustrated in FIG. 2.

FIG. 2 is a diagram for describing a schematic configuration of the diagnosis system according to an embodiment of the present disclosure.

Referring to FIG. 2, the diagnosis system 100 may include elements, such as those illustrated in FIG. 2.

The diagnosis system 10 may include a processor 110 and a storage device 120. The diagnosis system 100 means a data processing system having the computation ability for implementing the technical spirit of the present disclosure. In general, an average expert of a field to which the technology of the present disclosure pertains may easily infer that the diagnosis system 100 may be implemented as any device capable of performing a specific service, such as a personal computer or a portable terminal, in addition to a data processing system accessible to a client over a network.

The processor 110 may mean a computation device capable of driving a program 121 for implementing the technical spirit of the present disclosure. The processor 110 may perform a diagnosis by using the program 121 and a neural network 123 defined by the technical spirit of the present disclosure. The neural network may be a convolution neural network, and may output a diagnosis result through a trained neural network when receiving bio data (e.g., an image).

The program 121 may mean software defined to train the neural network 123 through supervised learning or to perform a diagnosis by using the trained neural network 123.

The storage device 120 may mean data storage means capable of storing the program 121 and the neural network 123. According to an implementation example, the storage device 120 may be implemented as a plurality of storage means. Furthermore, the storage device 120 may be a meaning including a temporary storage device or a memory which may be included in the processor 110, in addition to a main storage device included in the diagnosis system 100.

The diagnosis system 100 has been illustrated as being implemented as any one physical device in FIG. 2, but an average expert of a field to which the technology of the present disclosure pertains may easily infer that a plurality of physical devices may be organically combined to implement the diagnosis system 100 according to the technical spirit of the present disclosure.

Hereinafter, in this specification, when it is said that a diagnosis system (e.g., 100) performs a given function, this may mean that a processor (e.g., 110) included in the diagnosis system (e.g., 100) performs the given function by using a program (e.g., 121).

In this specification, when it is said that the diagnosis system 100 performs a diagnosis, this may mean a series of processes of receiving bio data and outputting output data defined in this specification, for example, a diagnosis result.

The diagnosis system 100 may receive bio data for each unit of a given unit. The unit of the given unit may be a pixel unit, a patch, or a slice unit, for example.

A diagnosis result of the diagnosis system 100 may be simply whether a disease has developed or a corresponding value (e.g., a probability) depending on the type of disease or may be state information indicating a degree of the state of a disease if a disease has developed.

For example, as will be described later, if the technical spirit of the present disclosure is used to diagnose prostate cancer, a Gleason pattern or a Gleason score, that is, an index indicative of a degree of the progress of prostate cancer, may be included in the state information. For example, the Gleason pattern has a value from 2 to 5. A higher value indicates that a degree that prostate cancer has developed is severe. Accordingly, the state information may include information corresponding to the probability that a biological tissue corresponding to the unit of a unit, that is, a target of diagnosis, may correspond to a specific value (e.g., 3, 4, or 5) or the Gleason pattern or information corresponding to the probability that the biological tissue may correspond to "normal" (i.e., if a disease has not developed).

In either case, the diagnosis system 100 may perform a function for classifying input data as a given diagnosis result through the trained neural network 123.

In order to classify input data as such a diagnosis result, according to an embodiment, the neural network 123 included in the diagnosis system 100 may output a given numerical value before determining the final diagnosis result as described above.

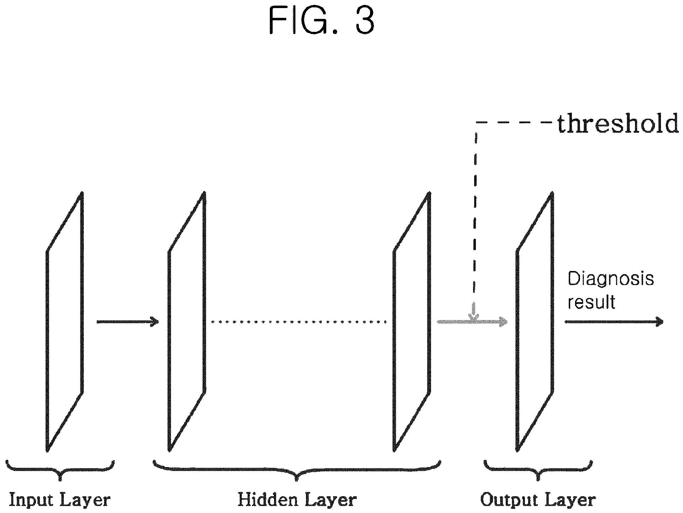

Such an example is illustrated in FIG. 3.

FIG. 3 is a diagram for describing a schematic diagnosis method of the neural network according to an embodiment of the present disclosure.

Referring to FIG. 3, as widely known, the neural network 123 may include an input layer, a hidden layer, and an output layer.

The output layer may output a diagnosis result, that is, a result that input data has been classified as one of predetermined classes. A layer prior to the output layer may be designed to output at least one numerical value. Accordingly, the output layer may determine a diagnosis result based on the numerical value, and may output the diagnosis result.

In this case, the numerical value may be a criterion for determining the diagnosis result. As described above, the numerical value may be a criterion for generating automated annotation training data according to an embodiment of the present disclosure. For example, when the numerical value is a first value or more, the neural network 123 may classify input data as a first diagnosis result. Furthermore, the input data may be included in automated annotation training data only when the numerical value among input data classified as the first diagnosis results is a second value or more.

In an embodiment, the first value and the second value may be identically set. In such a case, a reference numerical value for classifying a diagnosis result and criterion for determining the automated annotation training data may be determined as same value i.e. the second value.

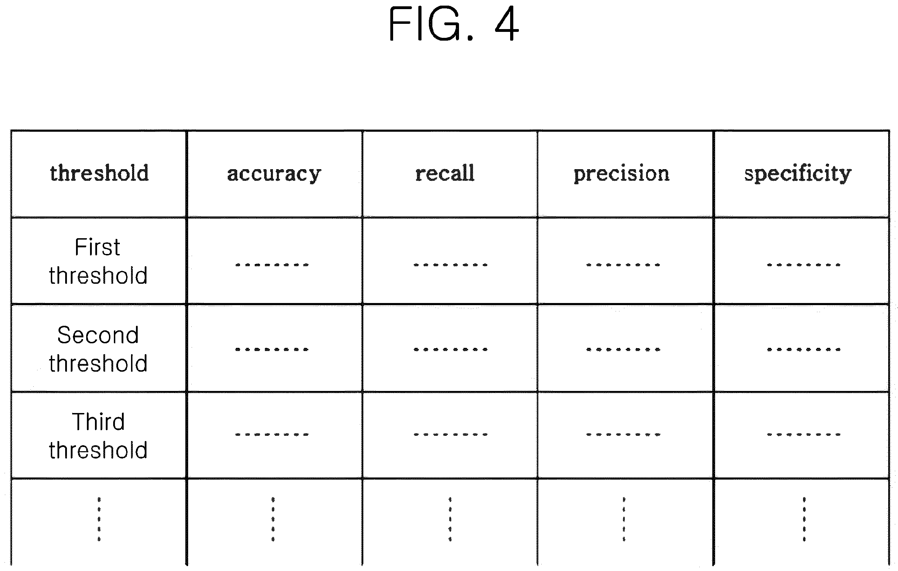

FIG. 4 is a diagram for describing a criterion for setting a reference threshold value according to an embodiment of the present disclosure.

Referring to FIG. 4, the diagnosis system 100 may specify a plurality of different automated annotation training data sets specified based on a plurality of threshold values.

In this case, the diagnosis system 100 may evaluate performance when the diagnosis system 100 is retrained by using each of the plurality of automated annotation training data sets.

The evaluation of performance may be performed based on at least one of representative criteria, that is, accuracy, precision, sensitivity, and specificity which evaluate performance of a learning model.

Performance may be determined to have been improved only when all the accuracy, precision, sensitivity, and specificity are improved. However, the evaluation of performance may be performed by placing emphasis on any one or some of the accuracy, precision, sensitivity, and specificity, if necessary.

The reason for this is that some of the accuracy, precision, sensitivity, and specificity may have a somewhat tradeoff relation depending on the type of input data or a design example of a neural network and the most important performance index among types of performance may be different depending on an embodiment.

If a plurality of criteria is included in an evaluation criterion, comprehensive performance may be evaluated depending on a predetermined weight factor.

When performance of each of the diagnosis systems 100 retrained using such a method is evaluated, a threshold value corresponding to automated annotation training data that produces the best performance may become a reference threshold value.

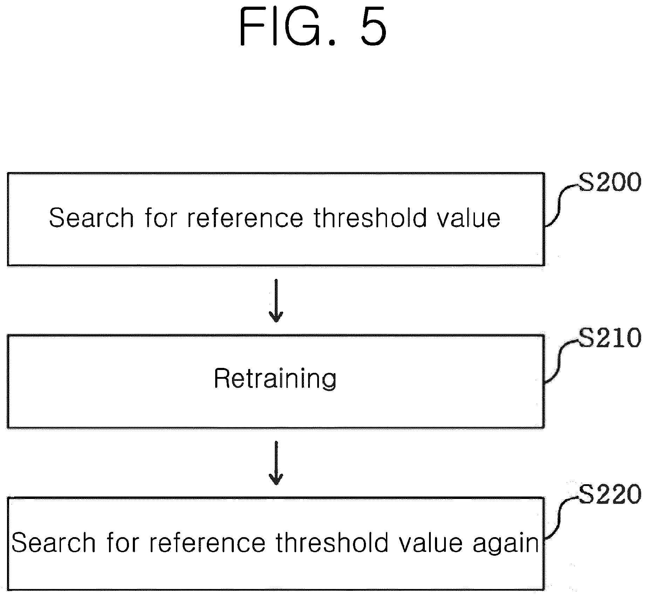

FIG. 5 is a diagram for describing a method of resetting a reference threshold value according to an embodiment of the present disclosure.

Referring to FIG. 5, as described above, when a reference threshold value is searched for and determined (S200), the diagnosis system 100 may generate a meaningful number of automated annotation training data previously determined based on the reference threshold value. In this case, the diagnosis system 100 may be a diagnosis system 100 retrained in order to determine a reference threshold value or may be a previous diagnosis system 100, that is, a system not retrained.

Furthermore, when a predetermined number of the automated annotation training data is generated, the diagnosis system 100 may perform retraining by using the generated automated annotation training data (S210). The automated annotation training data may need to satisfy a condition in which a predetermined number of data or the number of data within a predetermined ratio range is present for each diagnosis (class) which may be classified as a diagnosis result.

A given number of diagnoses according to new input data may be performed using the diagnosis system 100 on which retraining has been performed. Furthermore, if the diagnoses are performed to the extent that a reference threshold value can be searched for again, as described above, a new reference threshold value may be searched for again and determined. Furthermore, if a specific number of automated annotation training data or more is generated using the new reference threshold value, the diagnosis system 100 can be improved to have better performance by performing retraining again.

The method of providing a diagnosis system using semi-supervised learning according to an embodiment of the present disclosure may be implemented in a computer-readable recording medium in the form of computer-readable code. The computer-readable recording medium includes all types of recording devices in which data readable by a computer system is stored. Examples of the recording medium may include a ROM, a RAM, a CD-ROM, a magnetic tape, a hard disk, a floppy disk, an optical data storage device. Furthermore, the computer-readable recording medium may be distributed to computer systems connected over a network, and a computer-readable code may be stored and executed in a distributed manner. Furthermore, functional programs, codes and code segments for implementing the present disclosure may be easily inferred by programmer of a technical field to which the present disclosure pertains.

Although the present disclosure has been described with reference to the embodiments illustrated in the drawings, the embodiments are only illustrative. A person having ordinary skill in the art will understand that various modifications and other equivalent embodiments are possible from the present disclosure. Accordingly, the true technical range of protection of the present disclosure should be determined by the technical spirit of the following claims.

### [Industrial Applicability]

The present disclosure may be used in a "method of providing a diagnosis system using semi-supervised learning and a diagnosis system using the same."

## Claims

1. A method of providing a diagnosis system using semi-supervised learning, comprising steps of:
receiving, by a diagnosis system trained through a supervised learning-based neural network, given input data and outputting a diagnosis result of the input data;
generating, by the diagnosis system, automated annotation training data comprising the input data annotated as the diagnosis result; and
performing, by the diagnosis system, a retraining process by using the generated automated annotation training data.

2. The method of claim 1, wherein the step of generating, by the diagnosis system, the automated annotation training data comprising the input data annotated as the diagnosis result comprises including the input data in the automated annotation training data when a numerical value which is based on the diagnosis result and indicates a probability of the diagnosis result is a given threshold value or more.

3. The method of claim 2, further comprising steps of:
testing performance of the diagnosis system after performing the retraining process while changing the threshold value; and
determining a reference threshold value based on the results of the test.

4. The method of claim 1, wherein:
the diagnosis system generates the automated annotation training data by using a reference threshold value, and performs the retraining process by using the generated automated annotation training data, and
the method of claim 1, further comprising steps of
changing the reference threshold value after performing the retraining process.

5. The method of claim 1, wherein:
the learning system outputs, as the diagnosis result, any one of a plurality of diagnoses comprising a first determination and a second determination with respect to the input data, and
the automated annotation training data comprises a predetermined number or more of input data annotated as a first diagnosis, and a predetermined number or more input data annotated as a second diagnosis.

6. The method of claim 1, wherein:
the input data is bio data, and
the diagnosis result is at least any one of classes according to whether a disease has developed or a progress state of the disease.

7. A computer program installed in a data processing apparatus and stored in a computer-readable recording medium for performing the method according to any one of claims 1 to 6.

8. A data processing system comprising:
a processor; and
a storage device in which a program is stored,
wherein the method according to any one of claims 1 to 6 is performed by the program executed by the processor.

9. A diagnosis system comprising:
a processor; and
a storage device in which a program executed by the processor is stored,
wherein the program is stored in the storage device, and
enables a neural network trained based on supervised learning to receive given input data and output a diagnosis result of the input data,
generates automated annotation training data comprising the input data annotated as the output diagnosis result, and
performs retraining process of the neural network by using the generated automated annotation training data.

10. The diagnosis system of claim 9, wherein the program
includes the input data in the automated annotation training data when a numerical value which is based on the diagnosis result and indicates a probability of the diagnosis result is a given threshold value or more,
tests performance of the diagnosis system after performing the retraining process while changing the threshold value, and
determines a reference threshold value based on the results of the test.

11. The diagnosis system of claim 10, wherein the program
generates the automated annotation training data by using a reference threshold value,
performs the retraining process by using the generated automated annotation training data, and
changes the reference threshold value after performing the retraining process.

12. The diagnosis system of claim 9, wherein:
the program outputs, as the diagnosis result, any one of a plurality of diagnoses comprising a first determination and a second determination with respect to the input data, and
the automated annotation training data comprises a predetermined number or more of input data annotated as a first diagnosis, and a predetermined number or more input data annotated as a second diagnosis.
